# EUROPEAN PATENT SPECIFICATION

(11) **EP 1 862 171 B1**
(45) Date of publication and mention of the grant of the patent: **18.04.2012**
(21) Application number: 06011553.2
(22) Date of filing: 02.06.2006
(51) Int. Cl.: A61K 36/254, A61K 36/258, A61K 36/296, A61P 25/28

(54) **A medicine composition for treating muscular atrophy and myasthenia gravis and method of preparing the same**
Arzneizubereitung zur Behandlung von muskulärer Atrophie und Myasthenia gravis und die Herstellung derselben
Composition pour le traitement de l'atrophie musculaire et myasthénie grave et son procédé de préparation

(43) Date of publication of application: 05.12.2007
(73) Proprietor: Hebei Yiling Medicine Research Institute Co., Ltd., Hi -Tech Development District Shijiazhuang 050035 (CN)
(72) Inventor: Wu, Yiling, Shijiazhuang 050035 (CN)
(74) Representative: Grünecker, Kinkeldey, Stockmair & Schwanhäusser

(56) References cited:
- WO-A1-97/18824
- CN-A- 1 057 572
- CN-A- 1 174 066
- CN-A- 1 183 980
- CN-A- 1 277 852
- CN-A- 1 342 467
- JIANG F ET AL: "Beneficial effect of ginseng root in SOD-1 (G93A) transgenic mice." JOURNAL OF THE NEUROLOGICAL SCIENCES. 1 NOV 2000, vol. 180, no. 1-2, 1 November 2000 (2000-11-01), pages 52-54, XP002404325 ISSN: 0022-510X
- LIAO BAISONG ET AL: "Neuroprotective effects of ginseng total saponin and ginsenosides Rb1 and Rg1 on spinal cord neurons in vitro" EXPERIMENTAL NEUROLOGY, vol. 173, no. 2, February 2002 (2002-02), pages 224-234, XP002404326 ISSN: 0014-4886
- WU HANG-YU ET AL: "Effects of Yizhi Capsule on Learning and Memory Disorder and beta-amyloid Peptide Induced Neurotoxicity in Rats." CHINESE JOURNAL OF INTEGRATIVE MEDICINE. JUN 2006, vol. 12, no. 2, June 2006 (2006-06), pages 137-141, XP001247177 ISSN: 1672-0415

## Description

### Field of Technology

This invention relates to a medicine composition for treating muscular atrophy and myasthenia gravis and a method of preparing the same; it belongs to the field of traditional Chinese medicine and its production technology.

### Background of Technology

Motor neuron diseases (MNDs), comprising amyotrophic lateral sclerosis (ALS), progressive spinal muscular atrophy (PSMA), progressive bulbar palsy (PBP) and primary lateral sclerosis (PLS), are caused by the extensive degeneration of upper and lower motor neurons in central nervous system. The symptoms are the decrease of muscle strength and the muscle atrophy, and the choking and coughing during eating, dysphagia, dyspnoea and death as the diseases progress. ALS with unknown etiological factor is one kind of chronic progressive degeneration diseases that selectively damages the anterior horn cells in the spinal cord, brainstem motor nucleus and pyramidal tract and has the signs of impairment of the upper and lower motor neurons. ALS is the most frequent type of the chronic motor neuron diseases. According to the western medicine, because the pathogenesis of the ALS is not clearly understood, there is no special therapy method except for the maintenance therapy, comprising a sufficient nourishment, improvement of symptoms and psychological support, in order to encourage the patients to fight against the diseases.

According to the theory of the traditional Chinese medicine (TCM), the muscular atrophy caused by ALS and any other diseases belongs to the category of the TCM flaccidity syndrome. The ancient Chinese had proposed a viewpoint, believing in that treatment for flaccidity should aim at *Yangming* Channel. Professor Li Renxian considered that the fundamental of this disease was the weakness of both the spleen and the kidney while the incidental was the stirring-up of endopathic wind of deficiency type and there was a pathological factor of blood vessel impotency. He thought that the therapeutic method should focus on invigorating spleen and strengthening kidney, calming the endopathic wind syndrome and relieving convulsion and spasm, promoting blood circulation to remove collateral obstruction; this method has got some curative effect with a modified invigorating-spleen-and-strengthening-kidney recipe formulated by himself. Professor Deng Tietao thought that the ALS symptom was caused by insufficiency of both the spleen and the kidney with phlegm and blood stasis. He prescribed a modified Decoction for Reinforcing *Middle Jiao* and Replenishing *Qi* for oral use and Radix Astragali injection for intravenous and acupuncture point injection to invigorate the spleen and strengthen the kidney for the patients with ALS, together with some supplementary treatments comprising steaming and washing the upper limb to resolve the phlegm, the enema to expel the accumulative stool, acupuncture, massage, alimento-therapy and psychotherapy. His method had some effects, but was not satisfactory.

### Description of Invention

One object of this invention is to provide a medicine composition for treating muscular atrophy and myasthenia gravis with good effect.

Another object of this invention is to provide a method of preparing the said medicine composition.

The inventor found in the clinical practice that there was no significantly curative effect when using a therapeutic method of regulating spleen and stomach only. Therefore, the inventor places emphasis on the meridian, especially on the *Du* Channel of Eight Extra-Channels, instead of the above-said traditional treatment mode. The inventor has investigated the pathogenesis of the muscular amyotrophy with the approach of Eight Extra-Channels and proposed a new concept for treatment through the approach of Eight Extra-Channels and the therapeutic principle of strengthening primordial energy, supporting flaccidity, nourishing blood and promoting granulation. Warming up extra *Yang,* replenishing essence of life, activating Eight Extra-Channels are emphasized and some effective herbs that can warm up and invigorate *Du* Channel and invigorate the mind among large number of the traditional Chinese herbs have been selected and formulated. The said medicine composition is thus invented.

The active ingredients contained in the medicine composition of this invention are extracted from the traditional Chinese herbs as follows: 1-10 parts by weight of Ginseng and 1-10 parts by weight of Epimedium; a peferable ratio is 2-10 parts by weight of Ginseng and 2-8 parts by weight of Epimedium; a further preferable ratio is 5 parts by weight of Ginseng and 5 parts by weight of Epimedium, or 5 parts by weight of Ginseng and 2 parts by weight of Epimedium, or 9 parts by weight of Ginseng and 7 parts by weight of Epimedium, or 3 parts by weight of Ginseng and 1 part by weight of Epimedium.

Ginseng used in the medicine composition of this invention comes from its dry root and rhizome, the Acanthopanax gracilistylu plant, its medical property in the sense of the traditional Chinese medicine is sweet, a little bit bitter and mild. Epimedium used in the medicine composition of this invention comes from the dry and above-ground parts of the Aceranthus Sagittatus S.et Z., Epimedium Sagittatum (Sieb.et Zucc.) Maxim, Epimedium wushanense T.S.Ying or Korean Epimedium herb, the Berberidaceae plant, its medical property in the sense of the traditional Chinese medicine is acrid, sweet and warm.

The medicine composition of this invention can be formulated into any pharmaceutically accepted dosage forms such as injection, capsule, pill, tablet or oral liquid according to the conventional formulation technology. The injection was a preferable dosage form.

The method of preparing the medicine composition of this invention comprises the following steps:
(1)Weighting the traditional Chinese herbs according to the given prescription thereof;
(2) Extracting Ginseng with an alcohol having an alcohol strength of 60 to 90% for 1-3 times, combining the resultant extracts, filtering and concentrating the said combined extracts to form a concentrated solution, adding purified water into the concentrated solution to dilute it and mixing it till to being uniform, then refrigerating the mixture and filtering it; introducing the diluted filtrate into a macroporous adsorptive resin column, and eluting the column in sequence with aqueous eluant and an alcohol having an alcohol strength of 70% to 90%, collecting eluate and removing the impurities, then concentrating the eluate and drying it to obtain a Ginseng extract;
(3) Decocting and extracting Epimedium for 2-5 times with water, with of weight ratio of water:Epimedium being 10-30 for each time, combining the resultant extracts, filtering and concentrating the said combined extracts to form a concentrated solution, adding an alcohol into the concentrated solution until the concentration of alcohol of the solution reaches 60% to 80%, refrigerating the solution and filtering it, concentrating resultant filtrate to form a concentrated solution, adding water into the concentrated solution and mixing it till to being uniform, then refrigerating the mixture and filtering it; introducing the resultant filtrate into a macroporous adsorptive resin column, and eluting the column in sequence with an aqueous eluant and an alcohol having an alcohol strength of 40% to 60%, collecting eluate and removing the impurities, then concentrating the eluate and drying it to obtain an Epimedium extract;
(4) Blending the mixture of the extracts obtained from Step (2) and Step (3) with conventional medicinal adjuvant to obtain the medicine composition.

In step (2), the resin column is eluted with ammonia water at a pH 8-9 and an alcohol having an alcohol strength of 20% respectively; and, in step (3), the resin column is eluted with purified water and an alcohol having an alcohol strength of 20% respectively.

In step (2), the steps of removing the impurities are conducted as follows:

A purified water is added into the resultant eluate to let alcohol strength reach 40% to 60%; the resultant solution is brought to pass through a neutral alumina column and the effluent is collected; an appropriate amount of active carbon is added into the effluent and then the latter is refluxed for 20 minutes; keeping the effluent hot and subject it to a suction filtration with a plate filter; the resultant filtrate is collected and concentrated to form a concentrated solution; the solution is refrigerated for at least 24 hours, preferably 24-36 hours, and filtered;

In step (3), the step of removing the impurities is conducted as follows: an appropriate amount of diatomaceous earth is added into the eluate and mixed till to being uniform; an anhydrous alcohol is used for extraction of it for 2-5 times; the extracts are combined and concentrated.

In step (3), during each decoction with water, the amount by weight of water added is 10-30 times, preferably 15-25 times to that of the herb, and the first extraction time is 0.5-2 hours, while the time for the each subsequent extraction ranges from 0.5-1 hour.

Any of AB-8 type resin, D101 type resin and DM-130 type resin is preferred as the macroporous adsorptive resin to be used in the column in Step (2).

Any of AB-8 type resin, NKA type resin and S-8 type resin is preferred as the macroporous adsorptive resin to be used in the column in Step (3).

A preferred embodiment of the method according to the invention comprises the following steps:
(1) The traditional Chinese herbs to be used are firstly cleaned and ground, and then weighted according to the given prescription;
(2) The Ginseng radix is extracted with an alcohol having an alcohol strength of 60% to 90% for 3 times; for the first time the Ginseng is extracted with an alcohol at an amount by weight of 8 to 12 times to that of the Ginseng for 1-2 hours; for the second time, the Ginseng is extracted with an alcohol at an amount by weight of 6 to 10 times to that of the Ginseng for 1-2 hours; for the third time, the Ginseng is extracted with an alcohol at an amount by weight of 6 to 10 times to that of the Ginseng for 0.5 -1 hour. The resultant extracts are combined and filtered while they are kept hot, then condensed under a reduced pressure, with the alcohol being recovered at the same time, to obtain a concentrated solution; then put the concentrated solution into a stainless steel barrel, purified water is added into the concentrated solution and mixed till to being uniform. The mixture is refrigerated for 24-36 hours, laid it aside till to the room temperature and filtered with plate filter; the plate filter is preferably cleaned with a small amount of water. The filtrate is diluted with purified water and kept ready for use. Introducing the resultant diluted filtrate into a macroporous adsorptive resin column, such as AB-8 type or any other suitable resin column, and cleansing the column with an ammonia water at a pH 8 -9 and with an alcohol at a strength of 20% respectively. The column is eluated with an alcohol at alcohol strength of 80% and the eluate is collected. Purified water is added into the eluate to obtain a solution having alcohol strength of 50%. Let this solution pass through a neutral alumina column and the effluent is collected and combined; the resin column is then cleansed with a small amount of alcohol at an alcohol strength of 50% and the effluents are combined and kept ready for use. An appropriate amount of active carbon is added into the mixture and then the latter is boiled up and refluxed for 20 minutes. Keeping the mixture hot and subject it to a suction filtration with a plate filter. The resultant filtrate is collected and concentrated to form a concentrated solution. The solution is refrigerated overnight. A clarification plate filtration is conducted for the solution and the filtered solution is dried under reduced pressure. Then a light-yellow white amorphous powder, i.e., the Ginseng extract, is obtained.
(3) The Epimedium is extracted for 2-5 times, preferably 4 times. The amount of water by weight added for each extraction is preferably 10-30 times to that of the Epimedium. For the first time, the Epimedium is extracted for 0.5-2 hours, and for 0.5-1 hour for each remaining times. The afore-mentioned extracts are combined, filtered when they are hot, and condensed under a reduced pressure to obtain a concentrated solution, then put it into a stainless steel barrel. An alcohol is added into the concentrated solution till it reaches an alcohol concentration of 70%. The solution is refrigerated, filtered and concentrated with the alcohol being recovered. Purified water is added into the concentrated solution and mixed till to being uniform. The mixture is refrigerated, filtered and kept ready for use. Let the Epimedium solution obtained above pass through an AB-8 type macroporous adsorptive resin column and be cleansed with purified water and an alcohol having an alcohol strength of 20% respectively. The column is eluated with an alcohol having alcohol strength of 50% and the eluate is collected. An appropriate amount of diatomaceous earth is added into the eluate and mixed it till to being uniform; an anhydrous alcohol is used for extraction of it for 2-5 times; the extracts are combined, concentrated under a reduced pressure with the alcohol being recovered, and dried. Then the light yellow powder obtained is the extract of Epimedium.
(4) Some conventional medicinal adjuvant and a buffer salts such as 1,2- propylene glycol, sodium dihydrogen phosphate and disodium hydrogen phosphate are added into the mixture of the extracts obtained in step (2) and step (3). Water for injection is then added into the mixture until the volume of this water-mixture solution reaches 10ml-20ml. The resultant solution is refrigerated after heat treatment and then filtered with microporous membrane and ultrafiltration membrane in turn. The filtrate is filled, packaged and sterilized to obtain a medicine composition for injection.

Similarly, the extracts obtained in Step (2) and (3) can be formulated into the proper dosage forms such as the capsule, pill, tablet and oral liquid according to the conventional formulation technology.

Medicine effect tests have proved that the medicine composition can antagonize an inhibition to the regeneration capacity of the nerve terminals caused by serum in patients having amyotrophic lateral sclerosis, promote the regeneration and restoration of the damaged nerve terminals, improve swimming endurance capacity of experimental animals, enhance muscle strength significantly, inhibit significantly the increase of creatine phosphate kinase activity in mice with the muscular fatigue, and protect the function of skeletal muscles.

The sweet and mild Ginseng, based on the mild properties, is mixed with the low dose of sweet and warm Epimedium in the medicine composition in this invention, which not only makes the medicine composition capable of facilitating strengthening the vital essence and primordial energy and nourishing the kidney-yang, but also makes the medicine composition not too warm and dry. Although the active ingredients comprised in the medicine composition according the invention are extracted from only two traditional Chinese herbs, but the efficacy is affirmative and the compatibility is rigorous. The medicine composition is a new, effective and safe traditional Chinese medicine for treating the muscular atrophy caused by the motor neuron diseases (comprising the amyotrophic lateral sclerosis, the progressive spinal muscular amyotrophy, the progressive bulbar paralysis and the primary lateral sclerosis), the progressive muscular dystrophy, the congenital myopathy and myasthenia gravis.

### Embodiments

The embodiments of the present invention are given by the following examples of preparation of the medicine composition in the dosage forms of injection, capsule, tablet and pill.

### Example 1

The medicine composition in this example is made from the two traditional Chinese herbs at the ratio by weight as follows: 5 parts by weight of Ginseng and 2 parts by weight of Epimedium.

The preparation steps are as follows:
(1) The afore-mentioned two traditional Chinese herbs are cleaned and ground, and then weighted according to the prescription.
(2) The Ginseng is extracted with an alcohol having alcohol strength of 70% for 3 times. For the first time, an alcohol at an amount of 10 times to that of the Ginseng is added to the Ginseng powder, the mixture is heated to boiling point and extracted at this temperature for 1 hour. For the second time, an alcohol at an amount of 8 times to that of the Ginseng is added to the Ginseng residue after first extraction, and the mixture is heated to the boiling point and extracted at this temperature for 1 hour. For the third time, an alcohol at an amount of 8 times to that of the Ginseng is added to the Ginseng residue after second extraction, and the mixture is heated to the boiling point and extracted at this temperature for 0.5 hour. The resultant three extracts are combined and filtered while it is hot, then condensed under a reduced pressure to obtain a concentrated solution, then put it into a stainless steel barrel. Purified water is added into the concentrated solution and mixed it till being uniform. The mixture is refrigerated for 24 hours, laid the refrigerated solution aside till it reaches room temperature, and filtered it with a plate filter. The plate filter is cleansed with a small amount of water. The filtrate is diluted with purified water and kept ready for use. Let the concentrated Ginseng solution obtained above pass through a pre-treated AB-8 type macroporous adsorptive resin column, and resin column is then cleansed with an ammonia water at pH 8 - 9 and with an alcohol having an alcohol strength of 20% respectively. The resin column is eluated with an alcohol having alcohol strength of 80%, and the eluate is collected. Purified water is added into the eluate till it reaches an alcohol strength of 50%. Let this solution pass through a neutral alumina column and the effluent is collected. The resin column is also cleansed with a small amount of alcohol having alcohol strength of 50%, the effluents are combined. An appropriate amount of active carbon is added into the combined effluent and then the latter is boiled up and refluxed for 20 minutes. A suction filtration with plate filter is performed when the combined effluent is hot. The resultant filtrate is collected and concentrated to form a concentrated solution. The solution is refrigerated overnight. A clarification plate filter is used for filtration of it and the filtrate is condensed and dried under a reduced pressure. Then the Ginseng extract is obtained.
(3) The Epimedium is extracted for four times. The amount of water added each time is 20 times by weight to that of the herb. For the first time, the Epimedium is extracted for I hour, and for 0.5 hour for the following times. The afore-mentioned extracts are combined and filtered when it is hot, then condensed under a reduced pressure to obtain a concentrated solution, then put it into a stainless steel barrel. An alcohol is added into the concentrated solution till it reaches alcohol strength of 70%. The solution is refrigerated, filtered and concentrated with the alcohol being recovered. Purified water is added into the concentrated solution and mixed till to being uniform. The mixture is refrigerated and filtered. Let the Epimedium solution pass through a AB-8 type macroporous adsorptive resin column and is cleansed with purified water and an alcohol at a strength of 20% respectively. The column is eluated with an alcohol at alcohol strength of 50% and the eluate is collected. An appropriate amount of diatomaceous earth is added into the eluate and mixed uniformly. An anhydrous alcohol is used for extraction of the mixture for three times. The extracts are combined, concentrated under a reduced pressure and dried with the alcohol being recovered. Then the extract of Epimedium is obtained.
(4) Some conventional medicinal adjuvant such as 1,2- propylene glycol, sodium dihydrogen phosphate and disodium hydrogen phosphate are added into the mixture of the extracts obtained in step (2) and step (3). Water for injection is then added into the mixture obtained in this step till the volume of the solution reaches 16ml. The resultant solution is heated and boiled up for 30 minutes, then laid it aside till it reaches room temperature, and refrigerated. The solution is then filtered with microporous membrane and ultrafiltration membrane in turn. The filtrate is filled, packaged and sterilized to obtain a medicine composition according to the invention for injection.

### Example 2

The medicine composition in this example is made from the two traditional Chinese herbs at the ratio by weight as follows: 5 parts by weight of Ginseng and 5 parts by weight of Epimedium.

The steps for preparation of the medicine composition in details are basically same as that stated in Steps (1) - (3) as stated in Example 1. Some conventional medicinal adjuvants are added and then the medicine composition in the form of capsule is obtained according to conventional formulation technology.

### Example 3

The medicine composition in this example is made from the two traditional Chinese herbs at the ratio by weight as follows: 9 parts by weight of Ginseng and 7 parts by weight of Epimedium.

The steps for preparation of the medicine composition in details are basically same as that stated in Steps (1) - (3) in Example 1. Some conventional medicinal adjuvants are added and then the medicine composition in the form of tablet is obtained according to conventional formulation technology.

The protective effect of the medicine composition according to this invention on the motor neurons cultured in vitro will be further explained with the following animal tests.

Objective: To observe protective effect of the medicine composition of this invention (JWL) in the form for injection on the motor neurons cultured in vitro.

Methods: The embryonic spinal cord motor neurons in mice were separated with the density centrifugation and primarily cultured. The glutamic acid was added to establish the model of excitatory amino acid excitotoxicity was established and evaluate the protective effect of the medicine composition for injection in this invention (JWL) on the motor neurons. The cellular viability was detected with MTT method. The NF-200 immunohistochemical staining and the image analysis were used for measuring the length of the stem of the neurites. The lactate dehydrogenase (LDH) in the culture supernatant was detected with biochemistry analyzer. The TUNEL positive neuron count was used for observing the apoptosis of cells.

### 1. Materials and methods

### 1.1 Experiment materials

### 1.1.1 Medicines

The said medicine composition in the form for injection was prepared according to the said method in this invention, 8ml/ampoul (with the crude medicine 0.94g /ml). Rilutek^{®} Riluzole was manufactured by Aventis company with the imported medicine registration number H200020006 and Batch Number:28097. It should be dissolved with 0.9%NaCl-0.01N HCl before use.

### 1.1.2 Reagents

L15 culture medium, tetramethylenediamine, parenzyme (1:250), polylysine, rabbit anti- NF-200 antibody, insulin, DAB developer, MTT and DMSO were bought from Sigma company. The equine serum and Laminin were provided by Gibco BRL Company. The fetal bovine serum was produced by Hyclone, and TUNEL kit was manufactured by Roche Molecular Biochemical. The biotin labeling diplo-anti and horse radish peroxidase labeling tri-anti were manufactured by Beijing Zhongshan Biologic Products Co., Ltd. The other reagents were made in China with analytical purity and available in the market.

### 1.1.3 Animals

The clean grade SD female pregnant mice with the duration of pregnancy between 10 and 14 days were provided by the Animal Center, Institute of Field Operation Surgery, Daping Hospital, the Third Military Medical University, Chongqing, China.

### 1.1.4 Instruments

Enzyme labeling instrument (DG3022A, Shanghai), Image analytical system (Image Pro Plus4.5, U.S.A.), Automated biochemistry analyzer (BechMan CX7, U.S.A.).

### 1.2 Methods

### 1.2.1 Primary culture of the embryonic spinal cord motor neurons in mice

The embryonic mice with the pregnancy of about 14 days were selected and the spinal cord below the brain stem plane was taken out, and cut into 1 to 2mm³ pieces after the spinal meninges and blood vessel were stripped. Then the small pieces were digested for 30 minutes with 0.125% pancreatic enzymes at 37°C, and passed through the 200 Mesh steel sieve. The filtered liquid was engrafted into centrifuge tube with pre-placed 1ml 4%BSA (bovine serum albumin), and centrifuged for 10 minutes (300g). The supernatant was removed, dissolved and precipitated with L15 culture medium, and then added into the 5ml centrifuge tube with pre-placed 1ml 6.8%Metrizamide, and centrifuged for 15 minutes (500g). The light colored liquid between two layers of liquid was suctioned out and centrifuged for 10 minutes (300g). The supernatant was removed, dissolved with L15 culture medium and then the single cell suspension of the neural cells in the anterior horn of spinal cord was obtained. The concentration of the suspension was adjusted to 4.5 × 10⁵/ml.The suspension was implanted into 24-hole and 96-hole culture plates, and cultivated in CO₂ incubator. The maintenance culture medium is used after 24 hours. After 48 hours, 12.5 u g/ml 5-Fu was added and suctioned out after 24 hours, and then the fresh maintenance medium was used for cultivation.

### 1.2.2 Control of Experiment conditions

After the motor neurons were cultivated for 72 hours, the 0.5%,1% and 5% liquid of the said medicine composition for injection in this invention diluted by the fresh culture medium was respectively added into the experiment group, Riluzole was added into the Rilutek group (with the final concentration 10 µ mol/L) and the same amount of the culture medium was added into the control group. The three groups were cultured for 24 hours simultaneously. The glutamic acid was added (the final concentration was 0.5mmol/L) for the excitatory amino acid excitotoxicity and after 24 hours all the indices were detected.

### Determination of cellular viability with MTT method:

After carefully removing the culture solution in the 96-hole plate with the suction pipe, 10 µ L 5mg/mL MTT solution was added into each hole. After 5% CO₂ humidification reaction at 37°C for 4 hours, the supernatant was removed and the dimethyl sulphoxide (DMSO) of 100 µ L for each hole was added. The plate was put on the miniature oscillator for 5 minutes' oscillation at the room temperature. After the colored products was completely dissolved and measured with the automated enzyme marking instrument, the wave length λ =570nm, the reference wave length λ =630nm are used for the determination and the optical density value (OD) was measured.

### NF-200 immunobistochemical staining:

The cultured cells were conventionally fixed and sealed on the 6th day, the rabbit anti- NF-200 monoclonal antibody (1 : 1000) was added, and after 24 hours (4°C), the biotin labeled di-anti- IgG (1 : 300) was added. Then 60 minutes (37 °C ) later, the horse radish peroxidase-labeled streptavidin-peroxidase (1 : 300) was added for 60 minutes' reaction (37°C). The 0.01 mol/LPBS was used for rinsing for 5 minutes between the afore-mentioned steps, three times in total. The DAB solution was used for 5 minutes' coloration, the alcohol for gradient dehydration, the xylene for transparency and the neutral gum for sealing. In the negative control group, PBS replacing NF-200 antiserum is used for immunohistochemical reaction. Two pieces cover slip were taken respectively and 50 NF-200 positive neurons were randomly selected on each cover slip. The length of the stem of the neurite was measured with Quantimet-a image analytical system.

### LDH detection:

The cultured cells in the 24-hole plate were taken out. The supernatant was carefully suctioned out, grouped and numerated. The activity of the lactate dehydrogenase activity (LDH) in the supernatant was detected with automated biochemistry analyzer.

### TUNEL detection:

The cultured cells were fixed conventionally and sealed for 30 hours with 0.3% H₂O₂/methanol. The 0.1%Triton-0.1% sodium citrate solution was added and laid on ice for 2 minutes. The terminal deoxynucleotidyl transferase (TdT) and the fluorescein-labeled dUTP were added for two hours incubation at 37°C. The horse radish peroxidase-coupled anti-fluorescein antibody was added for 2 hours incubation at 37°C. The NBT/BCIP was used for coloration. The 0.01 mol/LPBS was used for rinsing for 5 minutes between the afore-mentioned steps, three times in total. The solution was dehydrated and sealed after it became transparent. The TdT enzyme was not added in the negative control group. The test was repeated three times for every experiment group. The number of positive neurons dyed with TUNEL was counted in five randomized fields of vision.

### Statistical analysis:

The data were expressed with mean and standard deviation ( x̅ ±s) . The t-test was used to compare the means each other. The analysis of variance was used to compare the significance of difference between groups.

### 2. Results

### 2.1 Morphology of spinal cord motor neurons

Four hours after the neurons were inoculated, cells began to adhere to the wall. The cell was single, and round or oval. The neurites were found in some motor neurons 24 hours later and the length of the neurite of motor neurons was significantly longer in 1% JWL group. After cultivated for 48 to 72 hours, the cells growing on the wall became obviously bigger; in cell body, the granular substance began to appear gradually; the circumference was clear; the neurite was increased in quantity and length. After 96 hours, the neurites were full in the fields of vision in a crisscross pattern. The motor neuron cells after growing 5 to 6 days were the most plump with better refraction. Nucleus located in the center or on one side of cells. Nucleus was bright in light color and could be easily distinguished from the perikaryon. Nucleus and nucleoli were evident. The neurites of the motor neurons in control group cultivated on the fifth day began to retract 24 hours after the glutamic acid was added. The cultured neurons of 1%JWL injection grew better with the suitable density. The neurites were big and thick and crossed over each other. In the glutamic acid control group, the apoptosis of motor neurons and more apoptosis body were found with TUNEL staining while in the said 1% JWL injection, there was significantly less apoptosis body.

### 2.2 The influence of the said medicine composition for injection in this invention on the normal motor neurons: refer to the Tablel( x̅ ±s)

There was difference in statistics sense in the cellular viability of motor neurons between the 0.5%,1% and 5% JWL groups and the control group (P<0.03). The cellular viability in the 1% JWL group was significantly better than that in the Rilutek group (P<0.05). The growth of neurites of the spinal cord motor neurons in the 0.5% and 1% JWL groups was significantly better than that in the control group (P<0.05) with the significant difference compared with Rilutek group (P<0.05).

**Table 1**

| Group | Concentration | OD value | Neurite (L, µ m) |
|---|---|---|---|
| Control group | | 0.230±0.130 | 159.71±48.95 |
| Rilutek group | 10umol/L | 0.266±0.141 | 192.08±89.07 |
| 0.5%JWL group | 4.5 µ g/L | 0.317±0.054 1 | 315.96±32.32 1,2 |
| 1%JWL group | 9 µ g/L | 0.396±0.087 1,2 | 373.46±80.24 1,2 |
| 5%JWL group | 45 µ g/L | 0.329±0.097 1 | 151.46±69.97 |

| | | | |
|---|---|---|---|
| Note: 1. Compared with the control group: P<0.05 2. Compared with Rilutek group: P<0.05 | | | |

### 2.3 The influence of the said medicine composition in the form for injection on the LDH leakage induced by the excitatory amino acid excitotoxicity for motor neuron: refer to Table2 (x̅ ±s) .

The detection result manifested that LDH in the culture supernatants of 0.5% JWL and of Rilutek groups were fewer than that in the control group (p<0.05) without significant difference (p>0.05) ,which suggested that both 0.5% JWL and Rilutek could decrease the LDH leakage of motor neurons caused by the glutamic acid damage.

**Table 2**

| Group | Concentration | LDH* |
|---|---|---|
| Glutamic acid control group | | 67.75±5.12 |
| Rilutek + glutamic acid group | 10umol/L | 58.50%4.20 1 |
| 0.5%JWL+glutamic acid group | 4.3µg/L | 39.00±4.971 |
| 1%JWL+ glutamic acid group | 9 µg/L | 61.50±3.11 |
| 5%JWL+ glutamic acid group | 43µg/L | 69.00±5.35 2 |

| | | |
|---|---|---|
| Note: 1. Compared with the control group: P<0.05 2. Compared with the Rilutek group: P<0.03 * Lactic dehydrogenase | | |

### 2.4 The influence of the said medicine composition in the form for injection on the motor neuron apoptosis caused by the excitatory amino acid excitotoxicity: refer to Table3 ( x̅ ±s).

The number of TUNEL positive cells in the 1%JWL group and the Rilutek group were fewer than that in the control group (p<0.05). The number of TUNEL positive cells in the 0.5 %JWL and 5%JWL group was decreased without significant difference compared with the control group (p>0.05), but with significant difference compared with the Rilutek group (p<0.05).

**Table 3**

| Group | Concentration | Number of TUNEL positive cells |
|---|---|---|
| Glutamic acid control group | | 19.8±5.63 |
| Rilutek + glutamic acid group | 10umol/L | 11.2±2.84 1 |
| 0.5%JWL+glutamic acid group | 4.5 µ g/L | 16.2±2.08 2 |
| 1%JWL+glutamic acid group | 9 µ g/L | 9.8±4.34 1 |
| 5%JWL+glutamic acid group | 45 µ g/L | 16.4±3.65 2 |

| | | |
|---|---|---|
| Note: 1. Compared with control group: P<0.05 2. Compared with Rilutek group: P<0.05 | | |

The afore-mentioned experiment indicates that the said medicine composition in this invention has the protective effect on both the normal motor neurons and the motor neurons damaged by the excitatory amino acid excitotoxicity.

## Claims

1. A medicine composition for use in treating muscular atrophy and myasthenia gravis, consisting of active ingredients extracted from traditional Chinese herbs as follows:
1-10 parts by weight of Ginseng and 1-10 parts by weight of Epimedium.

2. The medicine composition for use according to Claim 1, **characterized in that** the active ingredients are extracted from the traditional Chinese herbs as follows:
2-10 parts by weight of Ginseng and 2-8 parts by weight of Epimedium.

3. The medicine composition for use according to Claim 2, **characterized in that** the active ingredients are extracted from the traditional Chinese herbs as follows:
5 parts by weight of Ginseng and 5 parts by weight of Epimedium; or
5 parts by weight of Ginseng and 2 parts by weight of Epimedium; or
9 parts by weight of Ginseng and 7 parts by weight of Epimedium; or
3 parts by weight of Ginseng and 1 part by weight of Epimedium.

4. The medicine composition for use according to any one of claims 1- 3, **characterized in that** the medicine composition further comprises conventional medicinal adjuvant, and is formulated into a dosage form for injection and of capsule, pill, tablet or oral liquid.

5. The medicine composition for use according to Claim 4, **characterized in that** the medicine composition is formulated into a dosage form for injection.

6. A method of preparing the medicine composition according to any one of claim 1-5, comprises the following steps:
(1) Weighting the traditional Chinese herbs according to the given prescription thereof;
(2) Extracting Ginseng with an alcohol having an alcohol strength of 60 to 90% for 1-3 times, combining the resultant extracts, filtering and concentrating the said combined extracts to form a concentrated solution, adding purified water into the concentrated solution to dilute it and mixing it till to being uniform, then refrigerating the mixture and filtering it; introducing the diluted filtrate into a macroporous adsorptive resin column, and eluting the column in sequence with aqueous eluant and an alcohol having an alcohol strength of 70% to 90%, collecting eluate and removing the impurities, then concentrating the eluate and drying it to obtain a Ginseng extract;
(3) Decocting and extracting Epimedium for 2-5 times with water at a weight ratio of water: Epimedium being 10-30 for each time, combining the resultant extracts, filtering and concentrating the said combined extracts to form a concentrated solution, adding an alcohol into the concentrated solution until the concentration of alcohol of the solution reaches 60% to 80%, refrigerating the solution and filtering it, concentrating resultant filtrate to form a concentrated solution, adding water into the concentrated solution and mixing it till to being uniform, then refrigerating the mixture and filtering it; introducing the resultant filtrate into a macroporous adsorptive resin column, and eluting the column in sequence with aqueous eluant and an alcohol having an alcohol strength of 40% to 60%, collecting eluate and removing the impurities, then concentrating the eluate and drying it to obtain an Epimedium extract;
(4) Blending the extracts obtained from Step (2) and Step (3) with conventional medicinal adjuvant to obtain the medicine composition.

7. The method according to claim 6, **characterized in that**, in step (2), the resin column is eluted with ammonia water at pH 8-9 and an alcohol having an alcohol strength of 20% respectively; and, in step (3), the resin column is eluted with purified water and an alcohol having an alcohol strength of 20% respectively.

8. The method according to Claim 7, **characterized in that**, in step (2), the steps of removing the impurities are conducted as follows:
A purified water is added into the resultant eluate to let alcohol strength reach 40% to 60%; the resultant solution is brought to pass through a neutral alumina column and the effluent is collected; an active carbon is added into the effluent and then the latter is refluxed for 20 minutes; keeping the effluent hot and subject it to a suction filtration with a plate filter; the resultant filtrate is collected and concentrated to form a concentrated solution; the solution is refrigerated for at least 24 hours and filtered;
In step (3), the step of removing the impurities is conducted as follows: an appropriate amount of diatomaceous earth is added into the eluate and mixed till to being uniform; an anhydrous alcohol is used for extraction for 2-5 times; the extracts are combined, mixed and concentrated.

9. The method according to claim 8, **characterized in that**, in step (3), during decoction with water, the amount of water by weight is 15-25 times to that of the herbs, and that the first extraction time is 0.5-2 hours, and the time for the subsequent extractions is 0.5-1 hour.

10. The method according to Claim 9, **characterized in that** the macroporous adsorptive resin for the column in step (2) is selected from any of AB-8 type resin, D101 type resin and DM-130 type resin; the macroporous adsorptive resin for the column in step (3) is selected from any of AB-8 type resin, NKA type resin and S-8 type resin.

11. The method according to Claim 10, **characterized in that**, the traditional Chinese herbs used in step (1) are firstly cleaned and ground, and then is weighted according to the given prescription thereof;
In step (2), the Ginseng is extracted with an alcohol having an alcohol strength of 60% to 90% for 3 times, for the first time the Ginseng is extracted with the alcohol at an amount by weight of 8-12 times to that of the Ginseng for 1-2 hours; for the second time, the Ginseng is extracted with the alcohol at an amount by weight of 6-10 times to that of the Ginseng for 1-2 hours; for the third time, the Ginseng is extracted with the alcohol at an amount by weight of 6-10 times to that of the Ginseng for 0.5-1 hour; the resultant extracts are combined and filtered while they are hot, and then condensed under a reduced pressure to obtain a concentrated solution; a purified water is added into the concentrated solution and mixed till to being uniform; the mixture is refrigerated for 24 to 36 hours, laid it aside till to the room temperature and then filtered with a plate filter; the filtrate is diluted with purified water; the said macroporous adsorptive resin column is AB-8 type, and the resin column is eluted with an alcohol having an alcohol strength of 80%;
In step (3), the Epimedium is extracted for 4 times; for the first time, the Epimedium is extracted for 0.5-2 hours and each of the following three times for 0.5-1 hour; the resultant extracts are mixed and filtered when they are hot, and then condensed under a reduced pressure to obtain a concentrated solution; an alcohol is added into the concentrated solution until the alcohol concentration of the solution reaches 70%; the said macroporous adsorptive resin column is AB-8 type and the resin column is eluted with an alcohol having an alcohol strength of 50%;
In step (4), 1,2- propylene glycol, sodium dihydrogen phosphate and disodium hydrogen phosphate are added into the mixture of the extracts obtained from step (2) and step (3); water for injection is then added into the solution until the volume of the solution reaches 10ml to 20ml; the resultant solution is refrigerated after a heat treatment, and then filtered with microporous membrane and ultrafiltration membrane in turn; the filtrate is filled, packaged and sterilized to obtain the medicine composition for injection.

12. Use of a composition consisting of active ingredients extracted from traditional Chinese herbs as follows:
1-10 parts by weight of Ginseng and 1-10 parts by weight of Epimedium,
for the preparation of a medicament for the treatment of muscular atrophy and myasthenia gravis.

13. The use according to Claim 12, wherein the medicine composition is **characterized in that** the active ingredients are extracted from the traditional Chinese herbs as follows:
2-10 parts by weight of Ginseng and 2-8 parts by weight of Epimedium.

14. The use according to Claim 13, wherein the medicine composition is **characterized in that** the active ingredients are extracted from the traditional Chinese herbs as follows:
5 parts by weight of Ginseng and 5 parts by weight of Epimedium; or
5 parts by weight of Ginseng and 2 parts by weight of Epimedium; or
9 parts by weight of Ginseng and 7 parts by weight of Epimedium; or
3 parts by weight of Ginseng and 1 part by weight of Epimedium.

15. The use according to any one of Claims 12-14, wherein the medicine composition further comprises a conventional medicinal adjuvant, and is formulated into a dosage form for injection and of capsule, pill, tablet or oral liquid.

16. The use according to Claim 15, wherein the medicine composition is formulated into a dosage form for injection.

## Patentansprüche

1. Arzneimittelzusammensetzung, für die Verwendung zur Behandlung von Muskelatrophie und Myasthenia gravis, bestehend aus Wirkstoffen, welche wie folgt aus traditionellen chinesischen Kräutern extrahiert werden:
1-10 Gewichtsanteile von Ginseng und 1-10 Gewichtsanteile von Epimedium.

2. Arzneimittelzusammensetzung für die Verwendung nach Anspruch 1, **dadurch gekennzeichnet, dass** die Wirkstoffe wie folgt aus den traditionellen chinesischen Kräutern extrahiert sind:
2-10 Gewichtsanteile von Ginseng und 2-8 Gewichtsanteile von Epimedium.

3. Arzneimittelzusammensetzung für die Verwendung nach Anspruch 2, **dadurch gekennzeichnet, dass** die Wirkstoffe wie folgt aus den traditionellen chinesischen Kräutern extrahiert sind:
5 Gewichtsanteile von Ginseng und 5 Gewichtsanteile von Epimedium; oder
5 Gewichtsanteile von Ginseng und 2 Gewichtsanteile von Epimedium; oder
9 Gewichtsanteile von Ginseng und 7 Gewichtsanteile von Epimedium; oder
3 Gewichtsanteile von Ginseng und 1 Gewichtsanteil von Epimedium.

4. Arzneimittelzusammensetzung für die Verwendung nach einem der Ansprüche 1-3, **dadurch gekennzeichnet, dass** die Arzneimittelzusammensetzung ferner ein konventionelles medizinisches Adjuvans umfasst, und als Darreichungsform zur Injektion und als Kapsel, Pille, Tablette oder orale Flüssigkeit formuliert ist.

5. Arzneimittelzusammensetzung für die Verwendung nach Anspruch 4, **dadurch gekennzeichnet, dass** die Arzneimittelzusammensetzung als Darreichungsform zur Injektion formuliert ist.

6. Verfahren zur Herstellung der Arzneimittelzusammensetzung nach einem der Ansprüche 1-5, welches folgende Schritte umfasst:
(1) Wiegen der traditionellen chinesischen Kräuter entsprechend der dafür angegebenen Vorschrift ;
(2) Extrahieren von Ginseng unter Verwendung eines Alkohols für 1-3 Male, wobei der Alkohol einen Alkoholgehalt von 60 bis 90% aufweist, Kombinieren der resultierenden Extrakte, Filtrieren und Konzentrieren der besagten kombinierten Extrakte um eine konzentrierte Lösung zu bilden, Hinzufügen von gereinigtem Wasser in die konzentrierte Lösung um sie zu verdünnen, und Mischen bis sie einheitlich ist, dann Kühlen der Mischung und Filtern der Mischung; Einführen des verdünnten Filtrats in eine makroporöse adsorptive Harzsäule, und Eluieren der Säule der Reihe nach mit einem wässrigen Elutionsmittel und einem Alkohol, welcher einen Alkoholgehalt von 60 bis 90% aufweist, Sammeln des Eluats und Entfernen der Verunreinigungen, dann Konzentrieren des Eluats und Trocknen um einen Ginsengextrakt zu erhalten;
(3) 2-5 Male Auskochen und Extrahieren von Epimedium mit Wasser bei einem Gewichtsverhältnis von Wasser : Epimedium von 10-30 bei jedem Mal, Kombinieren der resultierenden Extrakte, Filtern und Konzentrieren der besagten kombinierten Extrakte um eine konzentrierte Lösung zu bilden, Hinzufügen eines Alkohols in die konzentrierte Lösung bis die Alkoholkonzentration der Lösung 60 bis 80% aufweist, Kühlen der Lösung und Filtern, Konzentrieren der resultierenden Filtrats um eine konzentriere Lösung zu bilden, Hinzufügen von Wasser in die konzentrierte Lösung und Mischen bis sie einheitlich ist, dann Kühlen der Mischung und Filtern der Mischung; Einführen des resultierenden Filtrats in eine makroporöse adsorptive Harzsäule, und Eluieren der Säule der Reihe nach mit einem wässrigen Elutionsmittel und einem Alkohol, welcher einen Alkoholgehalt von 40 bis 60% aufweist, Sammeln des Eluats und Entfernen der Verunreinigungen, dann Konzentrieren des Eluats und Trocknen, um einen Epimediumextrakt zu erhalten;
(4) Vermengen der aus Schritt (2) und (3) gewonnenen Extrakte mit einem herkömmlichen medizinischen Adjuvans, um die Arzneimittelzusammensetzung zu gewinnen.

7. Verfahren nach Anspruch 6, **dadurch gekennzeichnet, dass** in Schritt (2) die Harzsäule mit Ammoniakwasser bei einem pH-Wert von 8-9 beziehungsweise einem Alkohol, welcher einen Alkoholgehalt von 20% aufweist, eluiert wird; beziehungsweise, dass in Schritt (3) die Harzsäule mit gereinigtem Wasser und Alkohol eluiert wird, wobei der Alkohol einen Alkoholgehalt von 20% aufweist.

8. Verfahren nach Anspruch 7, **dadurch gekennzeichnet, dass** in Schritt (2) die Schritte zur Entfernung der Verunreinigungen folgendermaßen durchgeführt werden:
Ein gereinigtes Wasser wird in das resultierende Eluat hinzugefügt, um einen Alkoholgehalt von 40% bis 60% zu erreichen; die resultierende Lösung wird durch eine neutrale Aluminiumoxid-Säule geleitet und der Durchfluss wird gesammelt; eine Aktivkohle wird zum Durchfluss hinzugefügt und dann wird Letzteres für 20 Minuten refluxiert; wobei der Durchfluss heiß gehalten wird und einer Saugfiltration mit einem Plattenfilter unterworfen wird; das resultierende Filtrat wird gesammelt und konzentriert um eine konzentrierte Lösung zu bilden; die Lösung wird für mindestens 24 Stunden gekühlt und gefiltert;
In Schritt (3) wird der Schritt zur Entfernung der Verunreinigungen folgendermaßen durchgeführt:, eine angemessene Menge von Diatomeenerde wird zu dem Eluat hinzugefügt und gemischt bis es einheitlich ist; ein wasserfreier Alkohol wird 2-5 Mal zur Extraktion verwendet; die Extrakte werden kombiniert, gemischt und konzentriert.

9. Verfahren nach Anspruch 8, **dadurch gekennzeichnet, dass** in Schritt (3) während des Auskochens mit Wasser, die Menge des Wassers, bezogen auf das Gewicht, das 15-25fache der Menge der Kräuter beträgt, und dass die erste Extraktionszeit 0,5-2 Stunden ist, und die Zeit für die darauffolgenden Extraktionen 0,5-1 Stunde ist.

10. Verfahren nach Anspruch 9, **dadurch gekennzeichnet, dass** das makroporöse adsorptive Harz für die Säule in Schritt (2) von einem beliebigen AB-8 Harztyp, D101 Harztyp und DM-130 Harztyp ausgewählt ist; das makroporöse adsorptive Harz für die Säule in Schritt (3) von einem beliebigen AB-8 Harztyp, NKA Harztyp und S-8 Harztyp ausgewählt ist.

11. Verfahren nach Anspruch 10, **dadurch gekennzeichnet, dass** die in Schritt (1) verwendeten traditionellen chinesischen Kräuter zuerst gereinigt und gemahlen werden, und dann entsprechend der dafür angegebenen Vorschrift gewogen werden;
In Schritt (2), wird der Ginseng mit Alkohol 3 Mal extrahiert, wobei der Alkohol einen Alkoholgehalt von 60% bis 90% aufweist, beim ersten Mal wird der Ginseng mit Alkohol, in einer Menge bezogen auf das Gewicht, die das 8-12 fache der Menge des Ginsengs beträgt, für 1-2 Stunden extrahiert; beim zweiten Mal wird der Ginseng mit Alkohol, in einer Menge bezogen auf das Gewicht, die das 6-10 fache der Menge des Ginsengs beträgt, für 1-2 Stunden extrahiert; beim dritten Mal wird der Ginseng mit Alkohol, in einer Menge bezogen auf das Gewicht, die das 6-10 fache der Menge des Ginsengs beträgt, für 0,5-1 Stunde extrahiert; die resultierenden Extrakte werden kombiniert und gefiltert während sie heiß sind, und dann unter reduziertem Druck kondensiert, um eine konzentrierte Lösung zu erhalten; ein gereinigtes Wasser wird in die konzentrierte Lösung hinzugegeben und gemischt bis sie einheitlich ist; die Mischung wird für 24 bis 36 Stunden gekühlt, zur Seite gestellt bis die Raumtemperatur erreicht hat und dann mittels einer Plattenfilter gefiltert; das Filtrat wird mit gereinigtem Wasser verdünnt; die besagte makroporöse adsorptive Harzsäule ist vom AB-8 Typ, und die Harzsäule wird mit einem Alkohol, welcher einem Alkoholgehalt von 80% aufweist, eluiert;
In Schritt (3) wird Epimedium 4 Mal extrahiert; beim ersten Mal wird der Epimedium für 0,5-2 Stunden und bei jedem der darauf folgenden drei Male für 0,5-1 Stunde extrahiert; die resultierenden Extrakte werden gemischt und gefiltert wenn sie heiß sind, und dann unter reduziertem Druck kondensiert, um eine konzentrierte Lösung zu erhalten; ein Alkohol wird in die konzentrierte Lösung hinzugegeben bis die Alkoholkonzentration der Lösung 70% erreicht; die besagte makroporöse adsorptive Harzsäule ist vom AB-8 Typ und die Harzsäule wird mit einem Alkohol, welcher einen Alkoholgehalt von 80% aufweist, eluiert;
In Schritt (4) werden 1,2-Propylenglykol, Natriumdihydrogenphosphat und Dinatriumhydrogenphosphat zu der Mischung aus den in Schritt (2) und Schritt (3) gewonnenen Extrakten hinzugeben; dann wird Wasser für die Injektion in die Lösung hinzugegeben bis das Volumen der Lösung 10 ml bis 20 ml erreicht; die resultierende Lösung wird nach einer Hitzebehandlung gekühlt, und dann im Wechsel mit einer mikroporösen Membran und einer Ultrafiltrationsmembran gefiltert; das Filtrat wird abgefüllt, verpackt und sterilisiert, um die Arzneimittelzusammensetzung zur Injektion zu erhalten.

12. Verwendung einer Zusammensetzung, die aus Wirkstoffen besteht, welche wie folgt aus traditionellen chinesischen Kräutern extrahiert werden:
1-10 Gewichtsanteile von Ginseng und 1-10 Gewichtsanteile von Epimedium,
für die Herstellung eines Medikaments für die Behandlung von Muskelatrophie und Myasthenia gravis.

13. Verwendung nach Anspruch 12, wobei die Arzneimittelzusammensetzung **dadurch gekennzeichnet ist, dass** die Wirkstoffe wie folgt aus den traditionellen chinesischen Kräutern extrahiert werden:
2-10 Gewichtsanteile von Ginseng und 2-8 Gewichtsanteile von Epimedium.

14. Verwendung nach Anspruch 13, wobei die Arzneimittelzusammensetzung **dadurch gekennzeichnet ist, dass** die Wirkstoffe wie folgt aus den traditionellen chinesischen Kräutern extrahiert werden:
5 Gewichtsanteile von Ginseng und 5 Gewichtsanteile von Epimedium; oder
5 Gewichtsanteile von Ginseng und 2 Gewichtsanteile von Epimedium; oder
9 Gewichtsanteile von Ginseng und 7 Gewichtsanteile von Epimedium; oder
3 Gewichtsanteile von Ginseng und 1 Gewichtsanteil von Epimedium.

15. Verwendung nach einem der Ansprüche 12-14, wobei die Arzneimittelzusammensetzung ferner ein herkömmliches medizinisches Adjuvans umfasst, und als Darreichungsform zur Injektion und als Kapsel, Pille, Tablette oder orale Flüssigkeit formuliert ist.

16. Verwendung nach Anspruch 15, wobei die Arzneimittelzusammensetzung als Darreichungsform zur Injektion formuliert ist.

## Revendications

1. Composition de médicament pour utilisation dans le traitement de l'atrophie musculaire et de la myasthénie grave, constituée d'ingrédients actifs extraits à partir d'herbes chinoises traditionnelles comme suit :
1 à 10 parties en poids de ginseng et 1 à 10 parties en poids d'Epimedium.

2. Composition de médicament pour utilisation selon la revendication 1, **caractérisée en ce que** les ingrédients actifs sont extraits à partir d'herbes chinoises traditionnelles comme suit :
2 à 10 parties en poids de ginseng et 2 à 8 parties en poids d'Epimedium.

3. Composition de médicament pour utilisation selon la revendication 2, **caractérisée en ce que** les ingrédients actifs sont extraits à partir d'herbes chinoises traditionnelles comme suit :
5 parties en poids de ginseng et 5 parties en poids d'Epimedium ; ou
5 parties en poids de ginseng et 2 parties en poids d'Epimedium ; ou
9 parties en poids de ginseng et 7 parties en poids d'Epimedium ; ou
3 parties en poids de ginseng et 1 partie en poids d'Epimedium.

4. Composition de médicament pour utilisation selon l'une quelconque des revendications 1 à 3, **caractérisée en ce qu'**elle comprend en outre un adjuvant médicinal conventionnel, et est formulée sous une forme posologique pour injection et en capsule, pilule, comprimé ou liquide à usage oral.

5. Composition de médicament pour utilisation selon la revendication 4, **caractérisée en ce qu'**elle est formulée sous une forme posologique pour injection.

6. Procédé pour préparer la composition de médicament selon l'une quelconque des revendications 1 à 5, comprenant les étapes suivantes consistant à :
(1) peser les herbes chinoises traditionnelles conformément à la prescription indiquée pour celles-ci ;
(2) extraire le ginseng avec un alcool ayant un taux d'alcool de 60 à 90 % 1 à 3 fois, combiner les extraits résultants, filtrer et concentrer lesdits extraits combinés pour former une solution concentrée, ajouter de l'eau purifiée à la solution concentrée pour la diluer et la mélanger jusqu'à uniformité, puis réfrigérer le mélange et le filtrer ; introduire le filtrat dilué dans une colonne de résine adsorbante macroporeuse, et éluer la colonne successivement avec un éluant aqueux et un alcool ayant un taux d'alcool de 70 % à 90 %, collecter l'éluat et éliminer les impuretés, puis concentrer l'éluat et le sécher pour obtenir un extrait de ginseng ;
(3) décocter et extraire l'Epimedium 2 à 5 fois à l'eau avec un rapport en poids eau/Epimedium de 10 à 30 à chaque fois, combiner les extraits résultants, filtrer et concentrer lesdits extraits combinés pour former une solution concentrée, ajouter un alcool dans la solution concentrée jusqu'à ce que la concentration d'alcool dans la solution atteigne 60 % à 80 %, réfrigérer la solution et la filtrer, concentrer le filtrat résultant pour former une solution concentrée, ajouter de l'eau à la solution concentrée et la mélanger jusqu'à uniformité, puis réfrigérer le mélange et le filtrer ; introduire le filtrat résultant dans une colonne de résine adsorbante macroporeuse, et éluer la colonne successivement avec un éluant aqueux et un alcool ayant un taux d'alcool de 40 % à 60 %, collecter l'éluat et éliminer les impuretés, puis concentrer l'éluat et le sécher pour obtenir un extrait d'Epimedium ;
(4) combiner les extraits obtenus dans l'étape (2) et l'étape (3) avec un adjuvant médicinal conventionnel pour obtenir la composition de médicament.

7. Procédé selon la revendication 6, **caractérisé en ce que**, dans l'étape (2), la colonne de résine est éluée avec de l'ammoniaque à un pH de 8 à 9 et un alcool ayant un taux d'alcool de 20 % respectivement ; et, dans l'étape (3), la colonne est éluée avec de l'eau purifiée et un alcool ayant un taux d'alcool de 20 % respectivement.

8. Procédé selon la revendication 7, **caractérisé en ce que** ,
dans l'étape (2), les étapes d'élimination des impuretés sont effectuées comme suit : de l'eau purifiée est ajoutée à l'éluat résultant pour laisser l'alcool atteindre un taux de 40 % à 60 % ; la solution résultante est mise à passer dans une colonne d'alumine neutre et l'effluent est collecté ; un charbon actif est ajouté dans l'effluent et ensuite ce dernier est porté au reflux pendant 20 minutes ; l'effluant est maintenu chaud et soumis à une filtration par aspiration avec un filtre à plaque ; le filtrat résultant est collecté et concentré pour former une solution concentrée ; la solution est réfrigérée pendant au moins 24 heures et filtrée ;
dans l'étape (3), l'étape d'élimination des impuretés est effectuée comme suit : une quantité appropriée de terre de diatomées est ajoutée à l'éluat et mélangée jusqu'à uniformité ; un alcool anhydre est utilisé pour l'extraction 2 à 5 fois ; les extraits sont combinés, mélangés et concentrés.

9. Procédé selon la revendication 8, **caractérisé en ce que**, dans l'étape (3), durant la décoction avec de l'eau, la quantité en poids d'eau est de 15 à 25 fois celle des herbes, et **en ce que** la première durée d'extraction est de 0,5 à 2 heures, et la durée des extractions subséquentes est de 0,5 à 1 heure.

10. Procédé selon la revendication 9, **caractérisé en ce que** la résine adsorbante macroporeuse pour la colonne dans l'étape (2) est choisie parmi l'une quelconque d'une résine de type AB-8, une résine de type D101 et une résine de type DM-130 ; la résine adsorbante macroporeuse pour la colonne dans l'étape (3) est choisie parmi l'une quelconque d'une résine de type AB-8, une résine de type NKA et une résine de type S-8.

11. Procédé selon la revendication 10, **caractérisé en ce que** les herbes chinoises traditionnelles utilisées dans l'étape (1) sont d'abord nettoyées et broyées, puis sont pesées en fonction de la prescription indiquée pour celles-ci ;
dans l'étape (2), le ginseng est extrait avec un alcool ayant un taux d'alcool de 60 % à 90 % 3 fois, la première fois le ginseng est extrait avec l'alcool en une quantité en poids représentant 8 à 12 fois celle du ginseng pendant 1 à 2 heures ; la deuxième fois le ginseng est extrait avec l'alcool en une quantité en poids représentant 6 à 10 fois celle du ginseng pendant 1 à 2 heures ; la troisième fois le ginseng est extrait avec l'alcool en une quantité en poids représentant 6 à 10 fois celle du ginseng pendant 0,5 à 1 heure ; les extraits résultants sont combinés et filtrés pendant qu'ils sont chauds, et ensuite condensés sous pression réduite pour que soit obtenue une solution concentrée ; de l'eau purifiée est ajoutée à la solution concentrée et mélangée jusqu'à uniformité ; le mélange est réfrigéré pendant 24 à 36 heures, laissé de côté jusqu'à avoir atteint la température ambiante et ensuite filtré avec un filtre à plaque ; le filtrat est dilué avec de l'eau purifiée ; ladite colonne de résine adsorbante macroporeuse est de type AB-8, et la colonne de résine est éluée avec un alcool ayant un taux d'alcool de 80 % ;
dans l'étape (3), l'Epimedium est extrait 4 fois ; la première fois, l'Epimedium est extrait pendant 0,5 à 2 heures et chacune des trois fois suivantes pendant 0,5 à 1 heure ; les extraits résultants sont mélangés et filtrés en étant chauds, puis condensés sous pression réduite pour que soit obtenue une solution concentrée ; un alcool est ajouté à la solution concentrée jusqu'à ce que la concentration d'alcool de la solution atteigne 70 % ; ladite colonne de résine adsorbante macroporeuse est de type AB-8, et la colonne de résine est éluée avec un alcool ayant un taux d'alcool de 50 % ;
dans l'étape (4), du 1,2-propylèneglycol, du dihydrogénophosphate de sodium et de l'hydrogénophosphate disodique sont ajoutés au mélange des extraits obtenus dans l'étape (2) et l'étape (3) ; de l'eau pour injection est ensuite ajoutée à la solution jusqu'à ce que le volume de la solution atteigne 10 ml à 20 ml ; la solution résultante est réfrigérée après un traitement à la chaleur, et ensuite filtrée avec une membrane microporeuse et une membrane d'ultrafiltration tour à tour ; le filtrat est chargé, conditionné et stérilisé pour que soit obtenue la composition de médicament pour injection.

12. Utilisation d'une composition constituée d'ingrédients actifs extraits à partir d'herbes chinoises traditionnelles comme suit :
1 à 10 parties en poids de ginseng et 1 à 10 parties en poids d'Epimedium,
pour la préparation d'un médicament destiné au traitement de l'atrophie musculaire et de la myasthénie grave.

13. Utilisation selon la revendication 12, dans laquelle la composition de médicament est **caractérisée en ce que** les ingrédients actifs sont extraits à partir d'herbes chinoises traditionnelles comme suit :
2 à 10 parties en poids de ginseng et 2 à 8 parties en poids d'Epimedium.

14. Utilisation selon la revendication 13, dans laquelle la composition de médicament est **caractérisée en ce que** les ingrédients actifs sont extraits à partir d'herbes chinoises traditionnelles comme suit :
5 parties en poids de ginseng et 5 parties en poids d'Epimedium ; ou
5 parties en poids de ginseng et 2 parties en poids d'Epimedium ; ou
9 parties en poids de ginseng et 7 parties en poids d'Epimedium ; ou
3 parties en poids de ginseng et 1 partie en poids d'Epimedium.

15. Utilisation selon l'une quelconque des revendications 12 à 14, dans laquelle la composition de médicament comprend en outre un adjuvant médicinal conventionnel, et est formulée sous une forme posologique pour injection et en capsule, pilule, comprimé ou liquide à usage oral.

16. Utilisation selon la revendication 15, dans laquelle la composition de médicament est formulée sous une forme posologique pour injection.
